# EUROPEAN PATENT APPLICATION

(11) **EP 4 537 749 A1**
(43) Date of publication of application: **16.04.2025**
(21) Application number: 23742157.3
(22) Date of filing: 06.06.2023
(51) Int. Cl.: A61B 5/0205, A61B 5/259, A61B 5/282, A61B 5/318, A61B 5/00, A61B 5/11

(54) **ADJUSTABLE PATCH DEVICE**

(30) Priority: 09.06.2022 PT 2022118038
(71) Applicant: Cough Monitoring Medical Solutions S.A., 2825-182 Caparica (PT)
(72) Inventor: JACINTO TECELÃO, Diogo Bernardo, 2970-577 SESIMBRA (PT); NETO MAGALHÃES FERREIRA ANDRADE, Miguel, 2120-050 SALVATERRA DE MAGOS (PT); CAETANO VALADAS, Filipe André, 2795-042 LINDA-A-VELHA (PT); DIAS LOPES, Alexandra Sofia, 2620-047 OLIVAL BASTO (PT); ALMEIDA E SOUSA DE ALMADA LOBO, Sara Beatriz, 2800-043 ALMADA (PT); TIAGO GOUVEIA, André, 2840-565 SEIXAL (PT); COELHO DOS SANTOS PEREIRA, João Pedro, 2830-585 BARREIRO (PT); ALVES DA SILVA, Pedro Miguel, 2925-718 AZEITÃO (PT)
(74) Representative: Monteiro Alves, Inês
(86) International application number: PCT/PT2023/050014
(87) International publication number: WO 2023/239250

(57) **Abstract**

This invention describes an adjustable patch device for collecting and monitoring physiological data, which comprises at least two control units connected by at least one flexible connection element (4), wherein each of the said control units comprises at least one physiological data collection element (3) and electrical circuits (2).

The adjustable patch device allows for precise adjustment of the position of the physiological data collection elements (3) on the user's body, regardless of the size and anatomy thereof, in order to maximize the quality of the collected physiological signals.

## Description

### Technical Field

This invention relates to measurement devices for the purpose of diagnosis and monitoring diseases, and describes an adjustable patch device for collecting and monitoring physiological data.

### Background of the Invention

In recent years, there has been a considerable increase in devices that allow for the collection of physiological data to continuously and non-invasively monitor the health status of users over several days.

These devices primarily use the acquisition of physiological data such as heart rate, muscle activity, skin perspiration level, or respiratory activity. These data may be analyzed for various purposes, such as detecting arrhythmias, monitoring physical exercise and rehabilitation exercises, monitoring respiratory health, monitoring stress levels, among others.

These devices, often wearable, are becoming increasingly miniaturized, lightweight, discreet, comfortable, and seamlessly integrate into users' daily activities. These characteristics are crucial for the widespread adoption of these devices in users' day-to-day lives.

In particular, the miniaturization of electronics has enabled the development of compact solutions in which the physiological data collection elements (such as electrodes or sensors) are contained within the device itself (typically at the periphery), which is then placed in contact with the user's body.

In these devices, the attachment between the user and the device (with the contained physiological data collection elements) is attained mainly through two methods:
- Using specialized adhesives (which are, for example, waterproof and resistant to user movement and sweat), resulting in devices commonly referred to as patches; or
- Using straps or fabric bands that secure the device, as seen in the straps of smartwatches used for physiological monitoring.

This shift in paradigm (where the physiological data collection elements are contained within the device itself) has eliminated the need for cables extending from the device to the user thereof.

The use of cables for physiological data collection posed a problem for the widespread adoption of continuous monitoring solutions in users' daily lives, mainly for two reasons:
- User movement during their daily activities (e.g., walking or exercising) caused cable movement, leading to the corruption of the physiological data with motion artifacts; and
- The presence of long cables increased the likelihood of unintentional tugs, resulting in the disconnection or poor contact between the physiological data collection elements and the user thereof.

Furthermore, advances in engineering have allowed for the development of increasingly comfortable physiological monitoring patch devices, particularly through mechanisms that allow the devices to adapt to the diverse morphologies of users' bodies (e.g., body curvatures caused by different body compositions).

This adaptation to different body shapes has primarily been attained through a degree of flexibility in the patch devices. This flexibility has been predominantly accomplished in two ways:
- Through flexible electronic circuits (Flexible Printed Circuit; FPC) covered or coated with flexible materials, facilitating the device's adaptation to the curvatures of the user's body; or
- Through multiple rigid (non-flexible) circuits connected by electrical cables (typically using FPC), whereby the area with the electrical cables has some flexibility, allowing for adjustments to the curvatures of the user's body.

However, despite the existence of patch devices with degrees of flexibility that enable adaptation to the body curvatures, to date, there are no solutions that allow to adjust the placement of one or more related physiological data collection elements on the user's body with precision (a capability found in devices where the interface between the user and the physiological data collection elements was attained using cables).

Such feature is essential for patch devices to be used by all types of users, regardless of their body size and shape (for example, a single patch device should allow the collection of high-quality physiological data from specific body regions in both babies and adults, as the bodies of such users have different sizes and morphologies).

In this context, this invention describes an adjustable patch device for the collection and monitoring of physiological data, which comprises at least two control units connected by at least one flexible connecting element. Each of the said control units includes at least one physiological data collection element and at least one electrical circuit. The physiological data collection elements enable the collection of physiological signals and are electrically connected to the electrical circuits. Furthermore, at least one of the electrical circuits enables conditioning, processing, storage, and/or transmission of the collected data (among other functions).

The adjustable patch device addressed by the invention allows for the precise adjustment of the positioning of the physiological data collection elements. The connection between the control units containing the physiological data collection elements is made through one or more flexible connecting elements that are covered by a layer of flexible coating. These said flexible connecting elements allow for adjusting the position of the physiological data collection elements to accommodate the various sizes and morphologies of user bodies.

### Prior Art

The state of the art reveals numerous relevant approaches developed with the purpose of providing devices for the collection of physiological data with some degree of inherent flexibility.

Prior art document CN209091392U describes a multi-parameter physiological signal monitoring system that comprises a patch device containing a main control compartment, a battery compartment, and a flexible interface element. The flexible interface element electrically connects the two compartments through an embedded cable. Both the flexible interface element and the embedded cable are flexible and may be bent.

Similarly, document CN206080511U refers to an electrocardiographic monitoring device, which comprises two sensors connected by a flexible connection tube. The flexible connection tube of the device contains a pushbutton, thus indicating that the connection between the two sensors is made through an FPC-type circuit.

The flexibility present in the flexible interface element and the connection tube allows the device to adapt to the inherent curvatures of bodies, making them more comfortable for the user thereof. However, these devices do not allow for a precise adjustment of the positioning of the physiological data collection elements.

Considering the configuration of the devices, adjusting the positioning of the physiological data collection elements for different types of users would be possible only through significant bends of the flexible interface elements/connection tubes. However, given that these flexible interface elements/connection tubes were not designed to withstand significant bends, adjusting the positioning of the data collection elements would not be possible for three distinct reasons:
- Bending the flexible interface element/connection tube would be quite difficult due to the thickness of its material and of the cable contained inside.
- Even if it were possible to bend the flexible interface element/connection tube, it would be difficult to maintain the positioning of the physiological data collection elements on the user's body (as the bending of the element/tube generates a tension that causes it to return to the resting position (extended)).
- The electrical traces contained in the cable would break and cease to function after many bends (due to the mechanical pressure exerted during bending), as the flexible interface element/connection tube was not designed to withstand significant bends.

The prior art document US10667711B1 refers to a lightweight wearable device for monitoring physiological data. This device includes two components: a flexible patch circuit with two physiological data collection elements (electrodes) embedded at its ends, and a reusable recorder that fits onto the above-mentioned flexible patch circuit.

The flexibility of the flexible patch circuit allows the device to adapt to the body curvatures of users and thus be highly comfortable. However, considering the device's configuration, it is not possible to adjust the positioning of the physiological data collection elements without significantly bending the flexible patch circuit (i.e., to bring the electrodes closer or farther apart at its ends). While it is mechanically possible to make this bend, the flexible patch circuit was not designed to be bent as it is covered by an adhesive that must be in full contact with the user's skin. If it were bent to bring the electrodes closer, the adherence to the user's skin would be compromised, impacting the duration of the monitoring period.

The prior art document US2012029309A1 describes a patch-type device for monitoring physiological signals containing an electrical circuit and at least two physiological data collection elements (in the form of electrodes). The body of the device contains at least one flexible portion.

The flexible portions of the device allow it to adapt to the curvatures of users' bodies, making it more comfortable. However, this flexibility does not allow for varying the position of the physiological data collection elements.

Similarly, the prior art document WO2021041961A1 describes a device for collecting and monitoring physiological signals, comprising sensors coupled to a flexible strap and a flexible extender. The said strap has a more flexible area and a less flexible area, wherein the more flexible area may be bent.

However, this flexibility is aimed solely at allowing the device to adapt to the different body morphologies of users, without allowing for a precise adjustment of the position of the physiological data collection elements.

Furthermore, the flexible extender allows for adjusting the positioning of a third physiological data collection point, but within a limited area. However, this third physiological data collection point serves only as a reference for the other physiological data collection points (common-mode noise removal) and is optional.

It may thus be concluded that the previously proposed patch devices, despite having a degree of flexibility, do not allow for a consistent and precise adjustment of the positioning of the physiological data collection elements.

### Brief Description of the Invention

This invention describes an adjustable patch device for collecting and monitoring physiological data.

The above-mentioned adjustable patch device for collecting and monitoring physiological data comprises:
a first control unit comprising a first physiological data collection element and a first electrical circuit;
a second control unit comprising a second physiological data collection element and a second electrical circuit;
a first flexible connection element that connects the first control unit to the second control unit;
wherein the first and second physiological data collection elements are electrically connected to the respective first and second electrical circuits;
wherein the first control unit, its respective physiological data collection element, and the respective electrical circuit are included in a first casing;
wherein the second control unit, its respective physiological data collection element, and the respective electrical circuit are included in a second casing;
and wherein the least one electrical circuit is configured to condition, process, store, and/or transmit the physiological signals collected by the first and second physiological data collection elements;
wherein each of the first and second physiological data collection elements is selected from the group consisting of at least one electrode or a sensor based on the interaction of electromagnetic or mechanical waves with the user's body;
wherein the first flexible connection element is selected from the group consisting of a flexible electrical cable and a flexible flat cable and is configured to adjust the positioning and distance between the first and second physiological data collection elements.

Consequently, the adjustable patch device for collecting and monitoring physiological data in this invention provides adaptability through the flexible connection element, allowing for precise variation in the position and distance between the points of collection of the desired physiological data.

This adaptability enables the adjustable patch device to collect physiological data from specific parts of the body with quality, regardless of the user's age and size.

### Technical Problem

The proper placement of a physiological data collection device on the user's body is essential to ensure that the data is acquired correctly and with maximum quality.

This need for the correct placement varies depending on the sensor and the type of physiological data to be collected and analyzed.

In devices that collect electrocardiogram (ECG) tracings, the placement of the physiological data collection elements (electrodes) is done with the aim of highlighting and maximizing the quality and amplitude of specific waves (e.g., the P wave). Therefore, their placement should be in specific regions of the user's chest area.

However, both the specific position where the physiological data collection elements are placed and their relative distance vary considerably depending on the user's body size (e.g., in users of different ages). It is thus necessary to ensure that the device may enable the adaptation of the position of the physiological data collection elements according to the different sizes of the users' chest region.

In the previously proposed patch device solutions from the prior art, the distance and positioning between the physiological data collection elements are constant. Therefore, a device that is optimized to collect the electrocardiographic signal from an adult would be completely unsuitable for a child, resulting in a low-quality signal that potentially does not highlight the target waves.

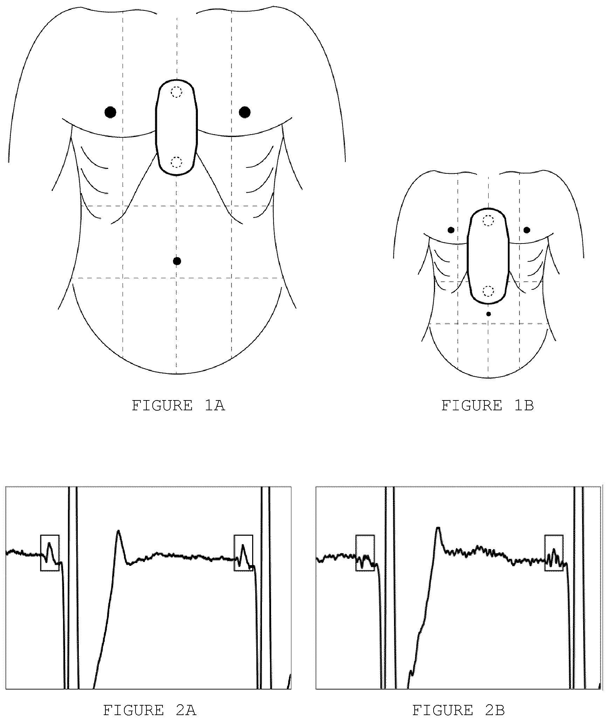

Figure 1 shows a prior art electrocardiogram signal collection device positioned on an adult user (Figure 1A) and a child (Figure 1B). As shown in Figure 1A, the device should be placed above the level of the chest region and in the epigastric region. However, when the same device is applied to a child (Figure 1B), the lower physiological data collection point ends up being placed in the mesogastric region, which will result in abnormal physiological data collection.

Figures 2A and 2B show the electrocardiogram signals obtained from the placement of the physiological data collection elements as indicated in Figures 1A and 1B, respectively. The signal shown in Figure 2A allows for high-quality visualization of the P wave (highlighted area in the respective Figures). In contrast, the signal shown in Figure 2B does not allow for reliable analysis of the P wave, which is of low quality due to the incorrect placement of the physiological data collection elements.

In devices that collect electromyography (EMG) signals for various applications, the placement of the physiological data collection elements (electrodes) should be done over the target muscles. However, the size of these muscles varies depending on the user's body size (e.g., in users of different ages).

Therefore, it is essential for the device to allow for the adaptation of the position of the data collection elements to ensure that data from the intended muscle(s) is collected.

As previously mentioned, in state-of-the-art solutions, the distance and positioning between the physiological data collection elements are fixed. Therefore, a device that is optimized to collect signals from a specific muscle in an adult would be completely unsuitable for a child, resulting in monitoring of other muscles (or a different anatomical part) than intended.

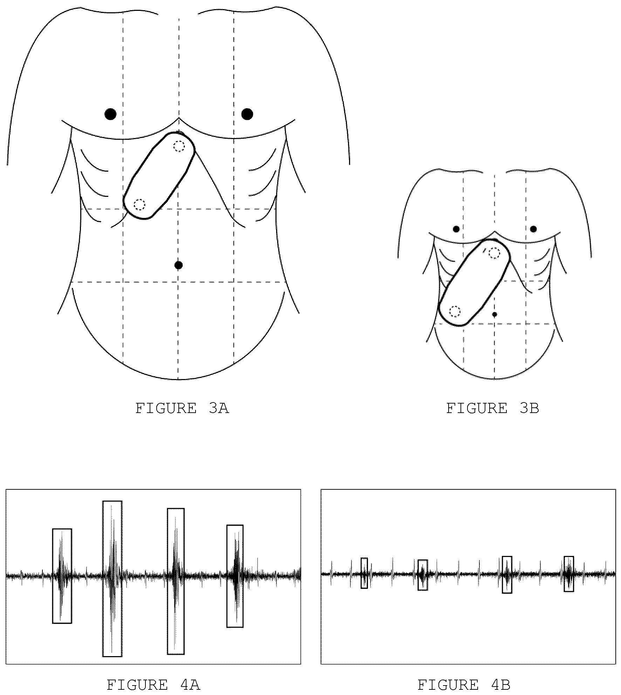

Figure 3 shows a state-of-the-art electromyography (EMG) data collection device for cough monitoring positioned on an adult user (Figure 3A) and a child (Figure 3B). As shown in Figure 3A, the device should be placed below the xiphoid process and on the periphery of the epigastric region (parallel to the ribs). However, when the same device is applied to a child (Figure 3B), the lower physiological data collection point ends up being placed in the lumbar region, resulting in abnormal physiological data collection.

Figures 4A and 4B show the electromyography signals caused by cough events obtained from the placement of the device as indicated in Figures 3A and 3B, respectively. The signal shown in Figure 4A allows for visualization of significant and well-defined muscle activations. In contrast, the signal shown in Figure 4B shows low-amplitude muscle activations and abnormal morphology due to the incorrect placement of the physiological data collection elements.

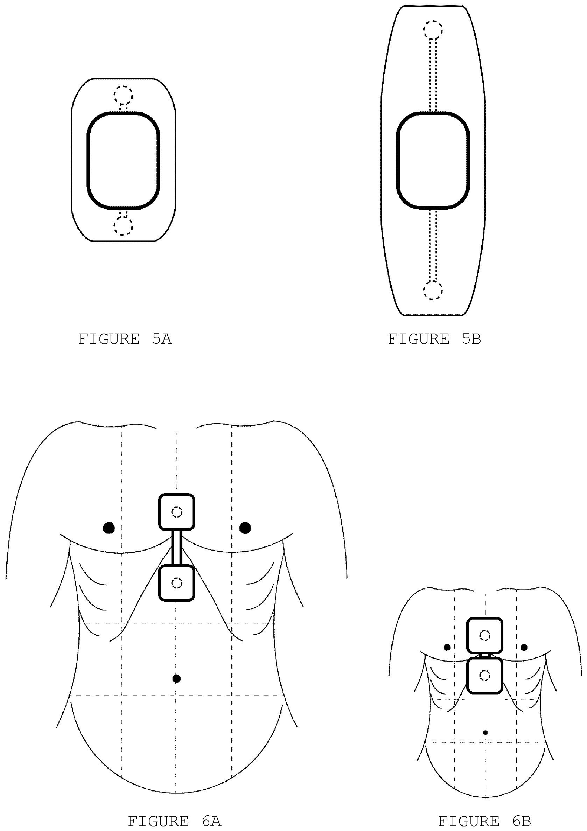

One possible solution to the described problem could be the manufacture of patches with different sizes and configurations (e.g., small size for children and large size for adults). Alternatively, the problem could also be remedied by using electrodes and adhesives with different sizes and configurations, which could be attached to fixed-size patches (like the devices constituting the prior art shown in Figures 5A and 5B).

However, these solutions end up being significantly more expensive to produce because each size (both of the device and the adhesive) is associated with a different production tool (e.g., different molds for each device size or different cutting tools for each adhesive size, respectively). Moreover, these solutions only allow for limited and discrete adjustment of the physiological data collection elements, lacking full adaptability and therefore not being optimized for intermediate sizes (e.g., there may be configurations available for infants and 16-year-old adolescents, but these configurations would not be optimized for an 8-year-old children).

### Solution to the Problem

This invention solves the problems at the state of the art by providing an adjustable patch device comprising at least two control units connected by at least one flexible connection element. Each of these control units comprises at least one physiological data collection element and at least one electrical circuit, whereby the physiological data collection elements are electrically connected to the electrical circuits. Furthermore, at least one electrical circuit is configured to condition, process, store, and/or transmit the physiological signals collected by the first and second physiological data collection elements.

The connection between the physiological data collection elements is mediated by at least one flexible connection element, which allows for precise adjustment of the positioning of these elements (according to the user's body size and anatomy).

Said flexible connection elements are resistant to sharp folds and allow for varying the configuration of the control units (and consequently, the physiological data collection elements) easily, comfortably, and conveniently, enabling the monitoring of physiological data from users of all types with maximum quality.

### Advantageous Effects of the Invention

This invention consists of an adjustable patch device that allows precise positioning of the physiological data collection elements on the user's body.

The interface between the device and the user thereof is attained through a mechanism selected from the group consisting of adhesives, gel electrodes (disposable or not), or suction mechanisms, all of which are comfortable and resistant to water and sweat. The flexibility of the adjustable patch device allows it to adapt to the user's body curvatures and be used ergonomically.

The adjustable patch device allows for the placement of physiological data collection elements to be easily adapted to different types of users. Therefore, the device may be applied to users of all ages, sizes, or anatomies (e.g., infants, adults, slim or obese individuals), enabling the extraction of physiological signals correctly and with maximum quality.

### Brief Description of the Figures:

In order to facilitate an understanding of the principles according to the embodiments of this invention, reference will be made to the illustrated embodiments in the Figures and the language used to describe them.

It should also be understood that there is no intention to limit the scope of the invention to the content of the Figures and that modifications to the inventive features illustrated herein, as well as additional applications of the illustrated principles and embodiments, which would normally occur to a person skilled in the art having possession of this description, are considered within the scope of the Claimed invention.
Figures 1A and 1B show a state-of-the-art patch device for collecting electrocardiogram traces, applied to an adult user and a child, respectively.
Figures 2A and 2B show the electrocardiogram signal resulting from the application of the state-of-the-art patch device as shown in Figures 1A and 1B, respectively.
Figures 3A and 3B show a state-of-the-art patch device for monitoring cough through electromyography signals, applied to an adult user and a child, respectively.
Figures 4A and 4B show the electromyography signal resulting from the application of the state-of-the-art patch device as shown in Figures 3A and 3B, respectively.
Figures 5A and 5B show a state-of-the-art patch device connected to small and large-sized adhesive patches, respectively, with built-in electrodes.
Figures 6A and 6B show the adjustable patch device addressed by this invention placed on an adult user (with the device in an extended position) and on a child (with the device in a contracted position), respectively.
Figures 7A and 7B show a top and internal view (without casing and flexible coating) of the adjustable patch device addressed by this invention in an extended and contracted configuration, respectively, representing the physiological data collection elements and standard connectors that connect the flexible connection element to the electrical circuits of each control unit.
Figures 8A and 8B show a side and internal view (without casing and flexible coating) of the adjustable patch device addressed by this invention in an extended and contracted configuration, respectively, representing the physiological data collection elements and standard connectors that connect the flexible connection element to the electrical circuits of each control unit.
Figures 9A and 9B show a top and external view (with casing and flexible connection element with coating) of the adjustable patch device addressed by this invention in an extended and contracted configuration, respectively.
Figures 10A and 10B show a side and external view (with casing and flexible connection element with coating) of the adjustable patch device addressed by this invention in an extended and contracted configuration, respectively.
Figures 11A, 11B, and 11C represent the degree of adaptability to the user's curvatures of the adjustable patch device addressed by this invention when placed in the abdominal region, indicating, respectively, (a) a good degree of adaptability, (b) a poor degree of adaptability, and (c) an excellent degree of adaptability to the user's curvatures.
Figures 12A and 12B show, respectively, a side view and a top view of the adjustable patch device addressed by this invention in a manner whereby the flexible coating of the flexible connection element has notches or grooves that allow for a slight curvature.
Figure 13 shows a side view of the adjustable patch device addressed by this invention in a manner whereby the flexible coating of the flexible connection element is produced with a more pronounced curvature.
Figure 14 provides a detailed view of the connection between the flexible coating of the flexible connection element and the casing.
Figures 15A and 15B show, respectively, a side view and a top view of the adjustable patch device addressed by this invention in a mode comprising a fastening element for the flexible connection element.
Figures 16A and 16B show, respectively, a side view and a top view of the adjustable patch device addressed by this invention in a manner whereby the device comprises a flexible connection element disposed perpendicular to the body plane of the user thereof, through a standard connector.
Figures 17A and 17B show, respectively, a side view and a top view of the adjustable patch device addressed by this invention in a manner whereby the device comprises a flexible connection element disposed perpendicular to the body plane of the user thereof, wherein a perpendicular auxiliary electrical circuit is electrically connected to the main electrical circuit.
Figures 18A, 18B, and 18C show the top view of the adjustable patch device addressed by this invention in a manner whereby the device comprises a flexible connection element disposed perpendicular to the body plane of the user thereof, in various positioning configurations.
Figures 19A and 19B show, respectively, the side view and the top view of the adjustable patch device addressed by this invention in a manner whereby the device comprises two flexible connection elements disposed perpendicular to the body plane of the user thereof.
Figures 20A, 20B, and 20C show the top view of the adjustable patch device addressed by this invention in a manner whereby the device comprises two flexible connection elements disposed perpendicular to the body plane of the user thereof, in various positioning configurations.
Figure 21 presents the top and internal view (without casing and flexible coating) of the adjustable patch device addressed by this invention in a manner whereby the physiological data collection elements may be adjusted by rotating around an axis.
Figure 22 presents the top and external view (with casing and flexible connection element with coating) of the adjustable patch device addressed by this invention in a manner whereby the physiological data collection elements are adjusted in different configurations through a rotational movement.
Figures 23A and 23B show the top view and the side view of the position orienting device present in the adjustable patch device addressed by this invention in a manner whereby the control units may be rotated in different configurations around an axis.
Figure 24 shows the side view of the interior of the adjustable patch device addressed by this invention in a manner whereby the control units may be rotated in different configurations around an axis.
Figure 25 shows a top view of the adjustable patch device addressed by this invention in a mode comprising three control units.
Figures 26A and 26B show the adjustable patch device addressed by this invention in a mode comprising four control units, adjusted and placed on an adult and a child, respectively.
Figure 27 shows the side view of the device for attaching gel electrodes.
Figure 28 shows the side view of the embodiment of the adjustable patch device addressed by this invention in a manner whereby the dry electrodes are embedded in the device itself.
Figure 29 shows the side view of the embodiment of the adjustable patch device addressed by this invention in a manner whereby the dry electrodes are attachable through a screw mechanism.
Figures 30A and 30B show, respectively, the top view and the side view of the interior of the adjustable patch device addressed by this invention, in a manner whereby the physiological data collection elements are arrayed in the control units in the form of an array.

### Description of Embodiments

This invention describes an adjustable patch device for collecting and monitoring physiological data, comprising at least two control units (1) connected by at least one flexible connection element (4). Each of the control units comprises at least one physiological data collection element (3) that allows the collection of physiological signals, and each of the control units comprises at least one electrical circuit (2) to which the physiological data collection elements are electrically connected. Furthermore, at least one electrical circuit (2) enables conditioning, processing, storage, and/or transmission of the collected data, among others.

The adjustable patch device addressed by this invention allows adjusting the position and placement of the physiological data collection elements (3) on the body of users of all types, regardless of their size and anatomy, in order to maximize the quality of the collected physiological signals.

In a preferred embodiment of the invention, the adjustable patch device comprises:
a first control unit (1) comprising a first physiological data collection element (3) and a first electrical circuit (2);
a second control unit (1) comprising a second physiological data collection element (3) and a second electrical circuit (2); and
a first flexible connection element (4), which electrically connects the first control unit (1) to the second control unit (1).

The above-mentioned first and second physiological data collection elements (3) are selected from the group consisting of at least one electrode or a sensor based on the interaction of electromagnetic or mechanical waves with the user's body.

In a preferred embodiment, the electrode is selected from the group consisting of a gel electrode or a dry electrode, and the sensor is selected from the group consisting of an oximetry sensor and a spectroscopy sensor.

In alternative embodiments of this invention, the adjustable patch device further comprises additional sensors based on electromagnetic or mechanical waves that are not in contact with the user's body. These sensors are selected from the group consisting of a microphone, an accelerometer, a gyroscope, a magnetometer, an inertial measurement unit, a goniometer, a pedometer, force sensors, pressure sensors, piezoresistive sensors, piezo capacitive sensors, light sensors, external temperature sensors, capacitive sensors, inductive sensors, and other.

The connection between the first control unit (1) and the second control unit (1) is made through at least one flexible connection element (4), such as a cable or a flexible flat cable, which is resistant to bending and is configured to adjust the positioning and distance between the first physiological data collection element (3) and the second physiological data collection element (3).

Furthermore, the connection between each of the flexible connection elements (3) and each of the control units is made through standard connectors. This connection through standard connectors should be protected to ensure that the flexible connection element (4) does not disconnect from the electrical circuits (2) during device handling (e.g., due to unintentional pulling).

This protection may be attained through locking mechanisms contained within the connector itself and/or through the implementation of other mechanisms external to the connector. An external mechanism may include securing the flexible connection element (4) to a structure (such as the electrical circuit (2) or the walls of the casings (5)) with a fixing polymeric material, such as acetic or neutral silicone.

The said flexible connection element (4) should be highly flexible, thin, and lightweight, as well as capable of withstanding repeated and constant bending movements (including bending angles up to 180°) without breaking the electrical tracks contained within it. In an alternative embodiment of this invention, the flexible connection element (4) comprises at least one layer of flexible coating (7), which extends to the respective terminal fitting positions of the first flexible connection element (4) of the first casing (6) and the second casing (6). The said layer of flexible coating (7) is configured to protect the flexible connection element (4) while allowing bending and maintaining it to adjust the position and configuration of the physiological data collection elements (3).

In a preferred embodiment of this invention, the flexible connection element (4) is selected from a flexible electrical cable and a flexible flat cable (FFC). In the preferred embodiment of this invention, the flexible connection element (4) is a flexible flat cable.

The adjustable patch device addressed by this invention allows for precise adjustment of the positioning of the physiological data collection elements through the movements permitted by the flexible connection element (4) (extension, contraction, twisting, among others) .

Figures 6A and 6B show the adjustable patch device addressed by this invention adjusted to collect physiological data from an adult and a child, respectively, solving the problem of improper placement of the physiological data collection elements (3) shown in Figure 1.

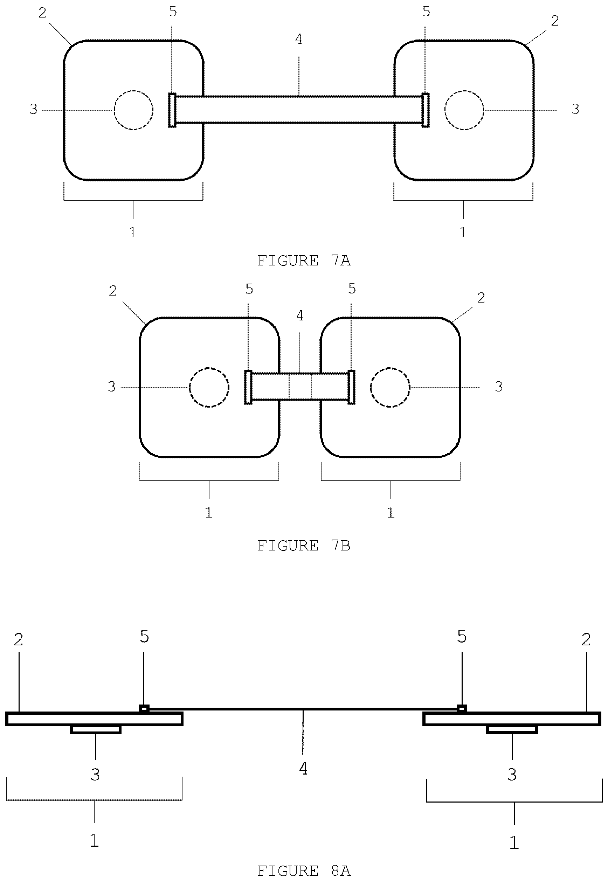

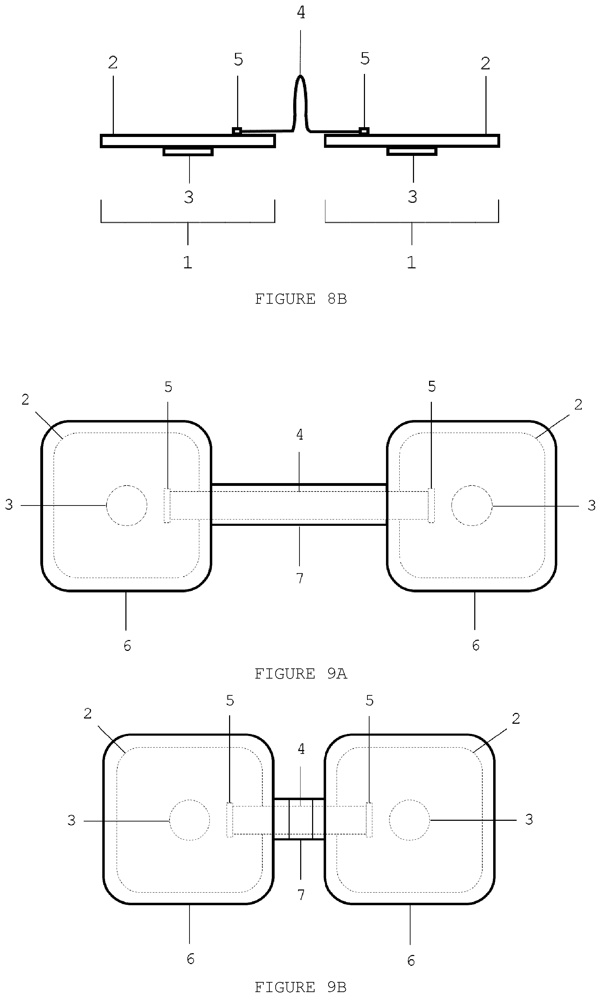

Figures 7A, 8A, 9A, and 10A show the adjustable patch device addressed by this invention in an extended position (i.e., whereby the distance between the physiological data collection elements (3) is maximum). Figures 7B, 8B, 9B, and 10B show the adjustable patch device addressed by this invention in a contracted position (i.e., whereby the distance between the physiological data collection elements (3) is smaller).

The electrical circuits (2) contain the connection to the physiological data collection elements (3) and all the electronics associated with the operation of the device, such as conditioning, processing, storage, and/or transmission of the collected data, among others. In a preferred embodiment, the electrical circuits (2) are connected to a power source and comprise, as a whole:
- physiological signal conditioning circuits, which vary according to the type(s) of physiological data being acquired by the physiological data collection elements (3) and monitored;
- a processing unit for controlling the device's operation; and
- a module for wireless communication with an external computing device and/or a memory module.

In a preferred embodiment, the communication module uses a communication protocol selected from the group consisting of USB, Bluetooth, Wi-Fi, and a mobile network.

All components of the electrical circuit (2) may be scattered throughout the various electrical circuits (2) of the device addressed by this invention.

The control units and their respective physiological data collection elements are included, each in a first and a second casing (6), respectively. In a preferred embodiment, the casings (5) are made of a rigid material selected from the group consisting of polycarbonate, polyethylene, polypropylene, or ABS (acrylonitrile-butadiene-styrene).

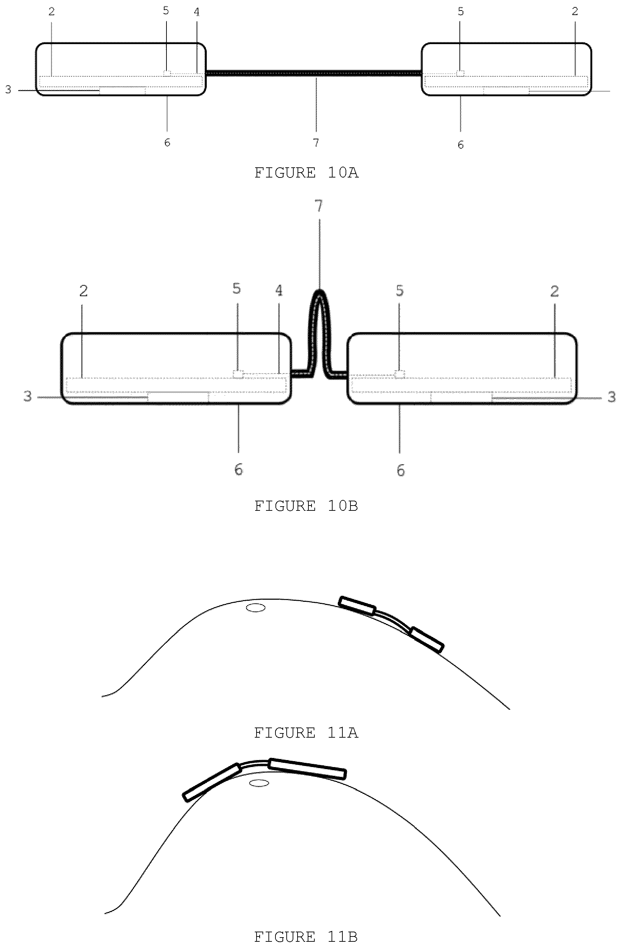

As shown in Figure 11A, the device in this embodiment allows for a good adaptation to the morphologies of certain types of users. However, for certain body morphologies (more irregular/pronounced), the adaptability provided by the rigid casings is lower, as shown in Figure 11B. In this case, the contact area between the rigid casings and the user's body is reduced, which may affect the collection of physiological data or the duration of monitoring.

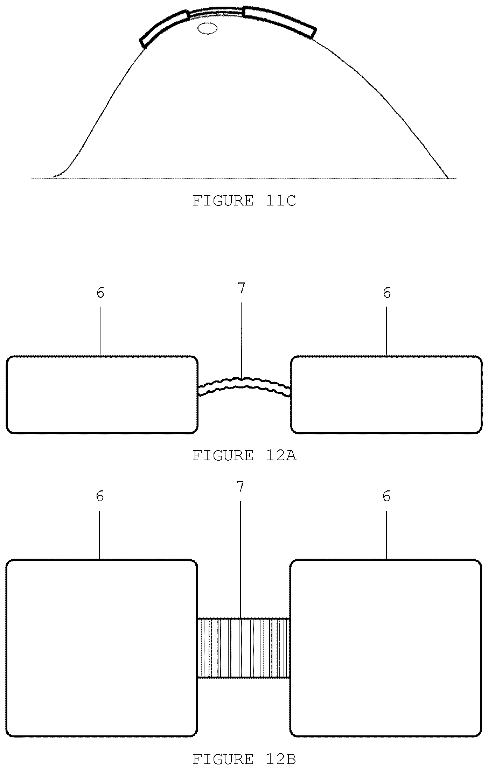

In an alternative embodiment, the casings (5) may be made of a flexible polymeric material, such as silicone or flexible rubber. In this embodiment, the entire device is flexible, as shown in Figure 11C.

In a preferred embodiment, the flexible electrical cable or flexible flat cable is coated with a likewise flexible material. Preferably, the flexible material of the casings (5) and the coating of the flexible coating (7) is a polymeric material with flexible properties, such as silicone or flexible rubber.

In this embodiment, the flexible coating (7) has sufficient thickness to provide resistance to user handling but allows for easy and natural bending. If it were too thick, folding would be difficult to perform and maintain. If it were too thin, the adjustable patch device would be quite fragile as the flexible coating (7) would tear easily.

Likewise, the morphological properties of the flexible coating (7) of the flexible connection element (4) are also essential for easy bending and maintaining that bend (i.e., the bending tension of the flexible coating (7) is significantly reduced). This is crucial for accurately adjusting the placement of physiological data on the user's body and maintaining that configuration throughout the monitoring period.

In a preferred embodiment, the flexible coating (7) of the flexible connection element (4) has a slight curvature, preferably with a 31.8mm arc.

Furthermore, the flexible coating (7) includes notches or grooves along its entire length to minimize bending tension and allow for pronounced curvatures (while the device is positioned and adjusted on a user). In a preferred embodiment, the notches or grooves are perpendicular to the length of the flexible connection element (4) and are arranged on the upper and lower planes of the flexible coating (7), alternating in the interface region between the flexible coating (7) and the casing (6). This embodiment is shown in Figure 12.

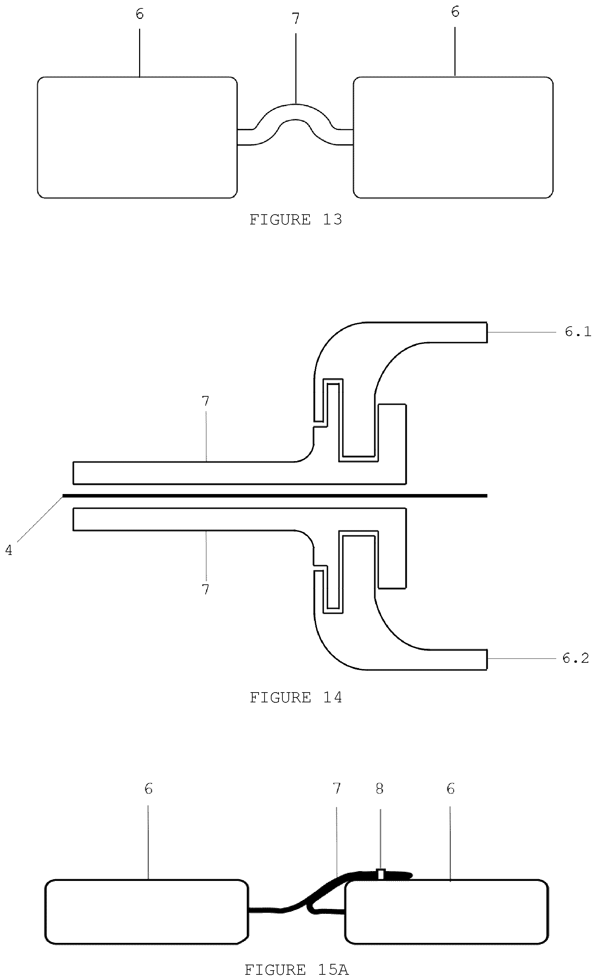

In another embodiment, the flexible coating (7) of the flexible connection element (4) is produced with a more pronounced curvature, facilitating material folding and reducing bending tension. This embodiment is shown in Figure 13.

In another embodiment (not shown in the Figures), the flexible coating (7) is configured like an accordion, wherein the flexible connection element (4) is glued to one of the walls of the flexible coating (7) and performs accordion-like movements and folds, together with the said flexible coating (7).

In the preferred embodiment of the invention, the flexible coating (7) is attached to the adjustable patch device by fitting it in the casings (5), as shown in figure 14. In the preferred embodiment, the rigid casings are comprised of an upper portion (6.1) and a lower portion (6.2).

In the preferred embodiment, the upper portion (6.1) and the lower portion (6.2) of their respective casings are welded or joined together by means of fitting elements, and the set composed by the casing (6) and the flexible coating (3) is sealed, thus preventing water from entering the control units (1). In the preferred embodiment, the adjustable patch device is water-resistant through the sealing attained by joining the upper (6.1) and lower (6.2) portions of the casing and the interface between the casing and the flexible coating (7).

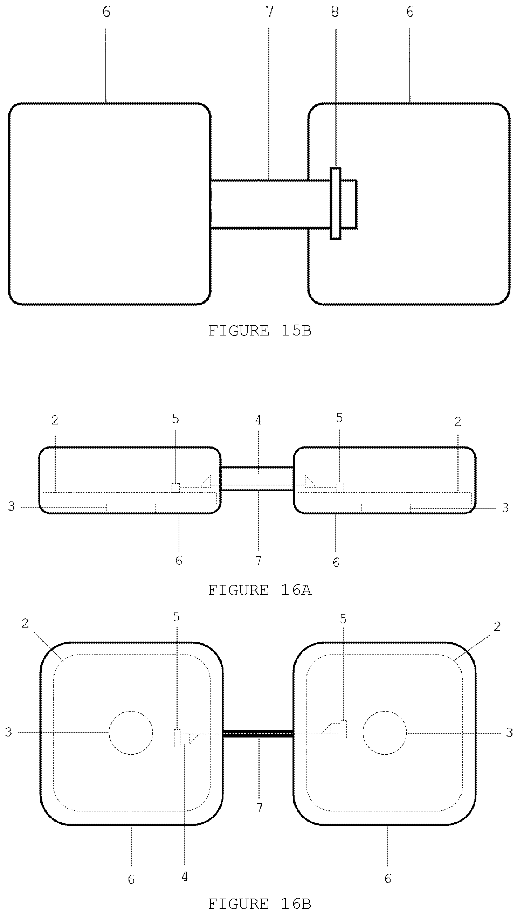

In an alternative embodiment, the flexible connection element (4) has a length such that when contracted, its fold causes a protrusion that may be uncomfortable for the user (e.g., becoming visible under clothing, rubbing against the user's body, and shaking during user movements). In this embodiment, the device addressed by this invention may optionally include a fastening element (7) configured to secure a portion of the flexible connection element (4) when it is in a contracted position, whereby this fastening element of the flexible coating (8) is situated on any face of the casing (6), preferably on the upper portion thereof (Figures 15A and 15B). In an alternative embodiment of this invention, the structure takes the form of a clip, into which the flexible cable or tape is inserted and secured.

In the preferred embodiment of the invention, the flexible connection element (4) is a flexible flat cable and is oriented parallel to the longitudinal plane that includes the respective control units to which the flexible connection element (4) is connected, i.e., the plane of the user's body. This arrangement allows the physiological data collection elements (3) to be brought closer together or moved apart, maximizing the device's adaptability to the user's body curvatures.

However, this embodiment only allows for adjusting the positioning of the physiological data collection elements (3) with a single degree of freedom. Depending on the application, it may be important to adjust the position of the physiological data collection elements (3) with multiple degrees of freedom (i.e., in various directions) .

Thus, in an alternative embodiment of this invention, the flexible connection element (4) is a flexible connection tape and is oriented orthogonally to the longitudinal plane that includes the respective control units to which the flexible connection element (4) is connected, i.e., perpendicular to the plane of the user's body, as shown in Figure 16.

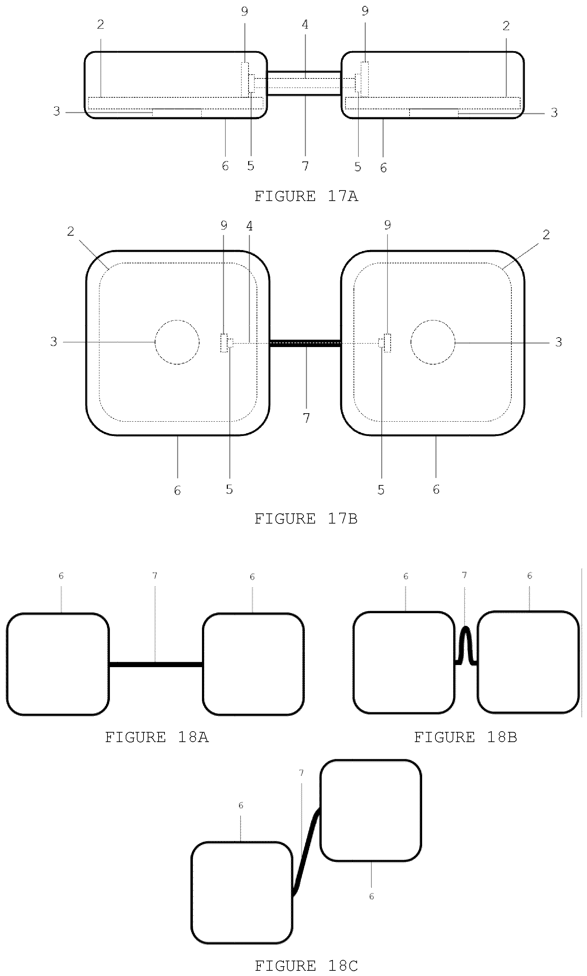

The orthogonal orientation of the flexible connection element (4) may be attained in two different ways: (i) by folding the flexible connection element (4) (as shown in Figure 16; this fold is only possible given the characteristics of the flexible connection element (4)) or (ii) by using a perpendicular auxiliary electrical circuit (9) electrically connected to the electrical circuit (2) through a standard connector (5) (as shown in Figure 17).

Figure 18 represents some of the possible positions that may be attained with this embodiment. However, since the flexible connection element (4) is disposed perpendicular to the plane of the user's body, the adaptation to the user's body curvatures will be limited.

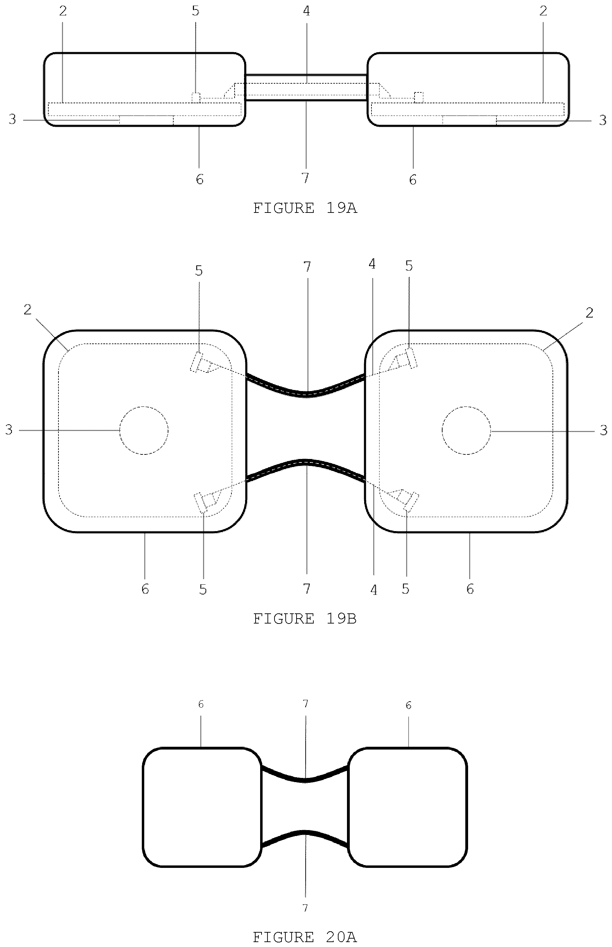

In another embodiment, the adjustable patch device may contain two or more symmetrical flexible connection elements (3) that establish electrical connection between the electrical circuits (2). In this embodiment, the flexible connection elements (3) are arranged perpendicular to the plane of the user's body, as shown in Figure 18; however, they may also be parallel to the plane of the user's body. This arrangement also allows for adjusting the position of the physiological data collection elements (3) along two axes, as shown in Figure 19.

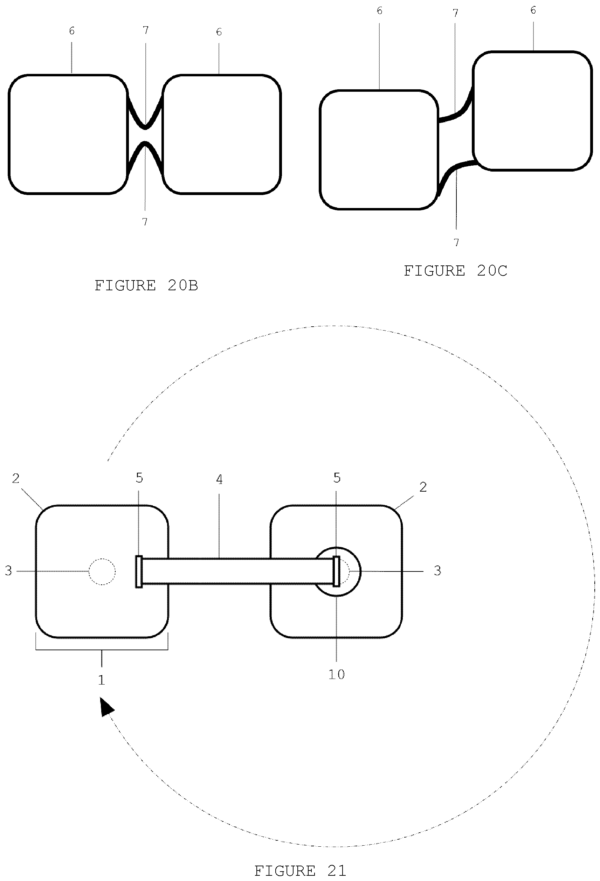

However, the arrangements shown in Figures 19 and 20 are not limiting, as the electrical connection between the two electrical circuits (2) may be attained through a greater number of flexible connection elements (3) and corresponding flexible coatings (7), arranged in various ways.

In a preferred embodiment of the invention, the device features two physiological data collection elements (3), arranged one per electrical circuit (2).

In an alternative embodiment of this invention, a greater and easier adjustment of the position of the physiological data collection element (3) may be attained by rotating the assembly that includes a control unit (1) / flexible connection element (4) around an axis located at the center of an adjacent control unit (1), in any of the embodiments involving different orientations of the flexible connection element (4) (Figure 21).

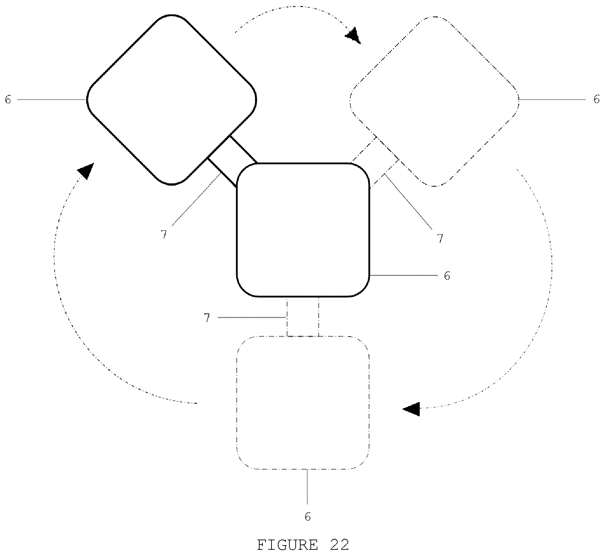

The possible configurations attained by the rotational movement are shown in Figure 22. The rotational movement is performed by a position-guiding device (10), which allows for the rotational movement while ensuring the connection between the respective electrical circuits (2).

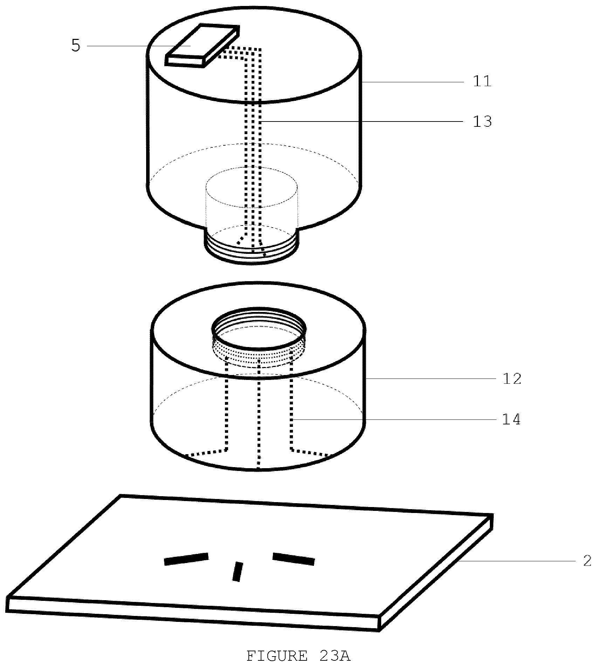

As seen in Figure 23A, the said position-guiding device (10) consists of a rotating element with a protrusion (11) (upper portion) and a fixed element with a recess (12) (lower portion). The fixed element with a recess (12) connects to the electrical circuit (2) contained in the control unit (1), while the rotating element with a protrusion (11) connects to the flexible connection element (4) through a standard connector (5).

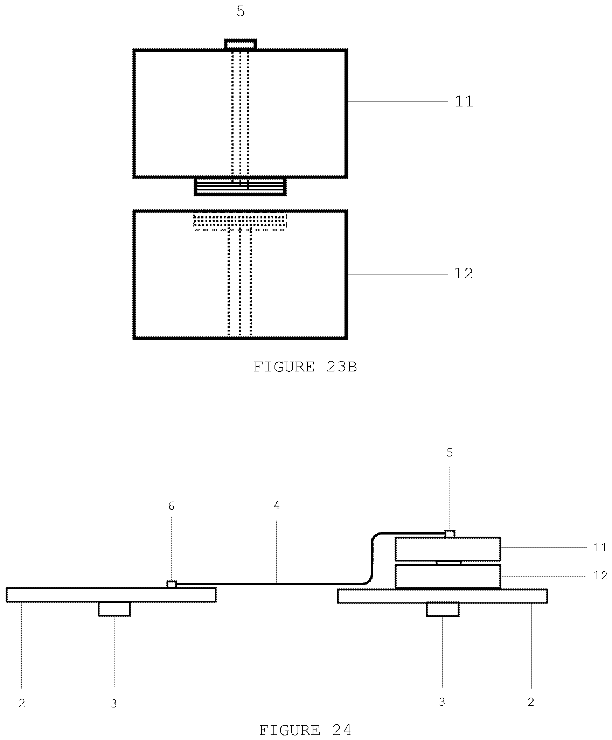

As shown in Figure 23B, the rotating element (11) fits into the fixed element (12) and rotates on it. This fit is attained through the protrusion on the lower portion of the rotating element (11) and the recess on the upper portion of the fixed element (12), wherein the said protrusion and recess have matching cylindrical shapes.

As shown in Figure 23, the standard connector (5) is electrically connected to the periphery of the protrusion of the rotating element (11) through a first set of electrical tracks (13), wherein the electrical tracks on the periphery of the protrusion take the form of rings arrayed in different transverse planes.

The recess of the fixed element (12), in turn, is connected to the electrical circuit (2) of the control unit (1) through a second set of electrical tracks (14), wherein the electrical tracks on the periphery of the recess also take the form of rings arrayed in different transverse planes.

The fitting of the protrusion of the rotating element (11) into the recess of the fixed element (12) results in the electrical connection between the rings of the first set of electrical tracks (13) and the rings of the second set of electrical tracks (14) along the different transverse planes. This electrical connection is maintained during the rotational movement of the rotating element (11) on the fixed element (12), ensuring the electrical connection between the associated control units.

Figure 24 shows the interior of the adjustable patch device in this embodiment of this invention.

In an alternative embodiment, the device may comprise more than two physiological data collection elements (3). In this embodiment, the above-mentioned electrical circuits (2) containing the physiological data collection elements (3) are arrayed in series and/or in parallel.

This embodiment is especially useful in certain applications. For example, the presence of a third physiological data collection element (3) allows its use as a reference to the first two physiological data collection elements (3) for common-mode noise removal purposes. This third collection point is typically placed on a bone where there is no electrophysiological activity. Furthermore, the use of multiple physiological data collection elements (3) is also beneficial for obtaining multiple perspectives of the same physiological event. For example, in a 12-lead electrocardiogram, ten physiological data collection elements (3) are used, resulting in 12 types of signals, with each signal providing optimized information about various relevant waves and intervals.

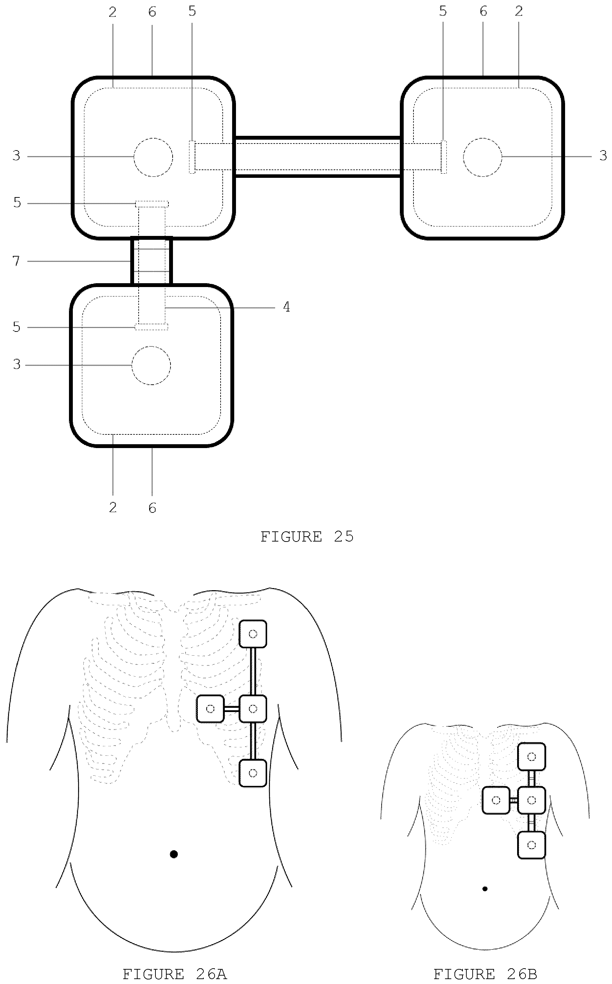

Figure 25 shows an adjustable patch device in this alternative embodiment, containing three physiological data collection elements (3). Figures 26A and 26B show the application of an adjustable patch device containing four physiological data collection elements (3), adjusted for an adult and a child, respectively (positioned to collect electrocardiogram data).

In an alternative mode of this invention, in the embodiment where the adjustable patch device contains more than two physiological data collection points, it is possible to configure which of the physiological data collection elements (3) collect physiological data and how their conditioning is done.

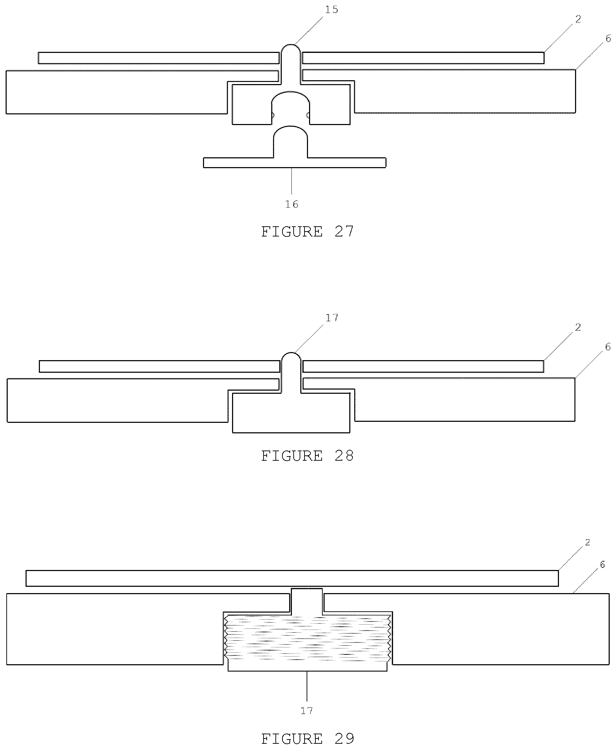

In a preferred embodiment of the invention, the physiological data collection elements (3) are gel electrodes (16). In this embodiment, the connection of the gel electrodes (16) to the electrical circuits (2) is done through an electrode connector (15), as shown in Figure 27. The said electrode connectors (15) may also be over-molded on the lower portions of the casing (6.2).

In another embodiment, the physiological data collection elements (3) are dry electrodes (17). In this embodiment, the electrodes are part of the device itself, as shown in Figure 28, or alternatively, the connection of the dry electrodes (17) to the electrical circuits (2) is done through a screw mechanism (as shown in Figure 29) or is over-molded onto the device.

In another embodiment, the connection of the dry electrodes (17) may be attained through a screw mechanism (as shown in Figure 29) or through magnets (not shown in the Figures), allowing them to be replaced when worn out (e.g., due to rusting). In this embodiment, any part of the dry electrode may make contact with the electrical circuits.

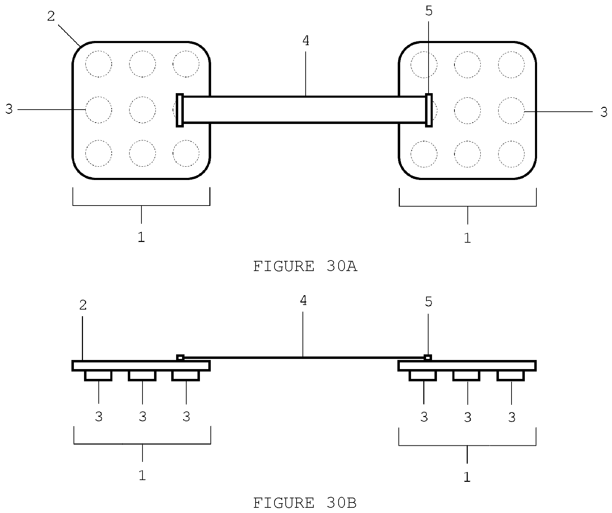

Furthermore, in alternative embodiments of this invention, the physiological data collection elements (3) may take various forms, composing an array of data collection elements within each electrical circuit, wherein the constituent points of the array are close enough to collect data from the same common or nearby region. This embodiment is shown in Figure 30.

This alternative embodiment may be particularly useful for removing artifacts from physiological signals that may contaminate the desired signal to be collected and for maximizing the quality of the constructed signal.

Finally, the attachment of the device to the user's body may be handled in three ways, which may be used individually or in combination: (i) through adhesive patches that stick to the user's skin on one side and to the device surface on the other side; (ii) through the gel electrodes themselves (16), which adhere to the user's skin on one side and attach to the device through specific connectors; and/or (iii) through suction mechanisms.

Furthermore, positioning the device on the user's body may be attained by (i) placing a first control unit (1) at the desired location and then placing a second control unit (1) through the extension or contraction of the flexible connection element (4), or (ii) bending the flexible connection element (4) into the desired configuration and then simultaneously placing the control units on the user's body.

The device is useful for monitoring physiological data such as electrocardiography, electromyography, electrogastrography, electrodermal activity, electroencephalography, body temperature, electrooculography, pulse oximetry, functional spectroscopy, among others.

The above-described subject matter is provided as an illustration of this invention and should not be interpreted in a limiting sense. The terminology used to describe preferred embodiments of this invention should not be construed to limit the invention to those particular embodiments.

As used in the description, definite and indefinite articles, in their singular form, are intended to encompass plural forms unless the context of the description explicitly indicates otherwise.

The indefinite articles "a" or "an" should generally be interpreted as "one or more" unless the sense of a singular embodiment is clearly defined in a specific situation.

It will be understood that the terms "comprise" and "include," when used in this description, specify the presence of stated features, elements, components, steps, and operations but do not preclude the presence or addition of one or more other features, elements, components, steps, and operations.

As used throughout this patent application, the term "or" is used in an inclusive sense rather than an exclusive sense unless the exclusive sense is clearly defined in a specific situation. In this context, a phrase such as "X utilizes A or B" should be interpreted as including all relevant inclusive combinations,

All changes, as long as they do not modify the essential characteristics of the following Claims, should be considered within the scope of protection of this invention.

### List of Reference Indications

1. Control unit
2. Electrical circuit
3. Physiological data collection element
4. Flexible connection element
5. Standard connector
6. Casing
6.1. Upper portion of the casing
6.2. Lower portion of the casing
7. Flexible coating
8. Flexible coating fixing element
9. Perpendicular auxiliary electrical circuit
10. Position orienting device
11. Rotating element with protrusion
12. Fixed element with recess
13. First set of electrical tracks
14. Second set of electrical tracks
15. Electrode connector
16. Gel electrode
17. Dry electrode

### List of Citations

The following is a list of citations:

### Patent Literature

CN209091392U
US10667711B1
CN206080511U
US2012029309A1
WO2021041961A1

## Claims

1. An adjustable patch device for physiological data collection and monitoring, **characterized in that** it comprises:
a first control unit (1) comprising a first physiological data collection element (3) and a first electrical circuit (2);
a second control unit (1) comprising a second physiological data collection element (3) and a second electrical circuit (2);
a first flexible connection element (4) electrically connecting the first control unit (1) to the second control unit (1);
wherein the said first and second physiological data collection elements (3) are electrically connected to said first and second electrical circuits (2);
wherein the first control unit (1) and its respective physiological data collection element (3) and electrical circuit are enclosed in a first casing (6);
wherein the second control unit (1) and its respective physiological data collection element (3) and electrical circuit are enclosed in a second casing (6);
wherein at least one electrical circuit (2) is configured to condition, process, store, and/or transmit the physiological signals collected by said first physiological data collection element (3) and second physiological data collection element (3);
wherein each of said first physiological data collection element (3) and second physiological data collection element (3) is selected from the group consisting of at least one electrode or a sensor based on the interaction of electromagnetic or mechanical waves with the user's body; and
wherein the first flexible connection element (4) is selected from the group consisting of a flexible electrical cable and a flexible flat cable and is configured to adjust the positioning and distance between the first physiological data collection element (3) and the second physiological data collection element (3).

2. The adjustable patch device according to Claim 1, **characterized in that** the first flexible connection element (4) comprises at least one layer of flexible coating (7), which extends to the respective terminal positions of attachment of the first flexible connection element (4) to the first casing (6) and the second casing (6) and is configured to protect the flexible connection element (4) while allowing its bending and maintenance to adjust the position of the physiological data collection elements (3).

3. The adjustable patch device according to either of the previous Claims, **characterized in that** the upper portion (6.1) and the lower portion (6.2) of the respective casings are welded or joined by means of fitting elements and the assembly formed by the casing (6) and the flexible coating (3) is sealed.

4. The adjustable patch device according to any of the previous Claims, **characterized in that** at least one of the first casing (6) and the second casing (6) has a fastening element (7) configured to secure a portion of the first flexible connection element (4) when disposed in a contracted position, wherein this flexible coating fastening element (8) is located on any face of the casing (6).

5. The adjustable patch device according to any of the previous Claims, **characterized in that** the electrical circuits (2) are connected to a power source and comprise:
- physiological signal conditioning circuits;
- a processing unit;
- a wireless communication module with an external computing device and/or a memory module.

6. The adjustable patch device according to Claim 1, **characterized in that** the physiological data collection element (3) is selected from the group consisting of a gel electrode (16) or a dry electrode (17), wherein said electrode is configured to collect electrical signals obtained through techniques selected from the group consisting of electrocardiography, electromyography, electrogastrography, electrodermal activity, electroencephalography, body temperature, and electrooculography.

7. The adjustable patch device according to Claim 1, **characterized in that** the sensors are selected from the group consisting of an oximetry sensor or a spectroscopy sensor.

8. The adjustable patch device according to any of the previous Claims, **characterized in that** it is comprised of additional sensors based on electromagnetic or mechanical waves, which are not in contact with the user's body.

9. The adjustable patch device according to Claim 1, **characterized in that** it is comprised of a third control unit (1) comprising a third physiological data collection element (3) and a second flexible connection element (4), which electrically connects the third control unit (1) to a control unit (1) selected from the group consisting of the first control unit (1) and the second control unit (1); and
wherein the third physiological data collection element (3) is selected from the group consisting of at least one electrode or a sensor based on the interaction of electromagnetic or mechanical waves with the user's body; and
wherein the second flexible connection element (4) is selected from the group consisting of a flexible electrical cable and a flexible flat cable and is configured to adjust a distance between the third physiological data collection element (3) and the first or second physiological data collection element (3).

10. The adjustable patch device according to Claim 9, **characterized in that** it is comprised of a fourth control unit (1) comprising a fourth physiological data collection element (3) and a third flexible connection element (4), which connects the fourth control unit (1) to the first control unit (1);
wherein the fourth physiological data collection element (3) includes at least one physiological data collection element (3) selected from the group consisting of at least one electrode or a sensor based on the interaction of electromagnetic or mechanical waves with the user's body; and
wherein the third flexible connection element (4) is selected from the group consisting of a flexible electrical cable and a flexible flat cable and is configured to adjust a distance between adjacent physiological data collection elements (3) when they are in contact with the user's body for physiological data collection.

11. The adjustable patch device according to any of the previous Claims, **characterized in that** at least one of the flexible connection elements (4) is a flexible flat cable, and the plane containing the at least one flexible connection element (4) is parallel or orthogonal to the longitudinal plane containing the respective control units to which the flexible connection element (4) is connected.

12. The adjustable patch device according to Claim 1, **characterized in that** the first casing (6) optionally comprises a position orienting device (10) configured to allow rotation of the assembly comprising a control unit (1) / flexible connection element (4) around an axis contained within the center of an adjacent control unit (1) when the said flexible connection element (4) is parallel or orthogonal to the longitudinal plane containing the respective control unit (1).

13. The adjustable patch device according to Claim 12, **characterized in that** the rotation guiding device consists of a rotating element with a protrusion (11) and a fixed element (12) with a recess, wherein the fixed element (12) is connected to the electrical circuit (2) contained in the control unit (1) and the rotating element (11) is connected to the flexible connection element (4) through a standard connector (5).

14. The adjustable patch device according to Claim 12 or 13, **characterized in that** the rotating element (11) fits into and rotates on the fixed element (12), wherein the fitting is attained through a protrusion on the bottom of the rotating element (11) and a recess on the top of the fixed element (12), wherein the said protrusion and recess have homologous cylindrical shapes.

15. The adjustable patch device according to any of Claims 12 to 14, **characterized in that** the standard connector (5) is electrically connected to the periphery of the protrusion of the rotating element (11) through the first set of electrical tracks (13), wherein the electrical tracks on the periphery of the protrusion take the form of rings arrayed in different transverse planes.

16. The adjustable patch device according to any of Claims 12 to 14, **characterized in that** the recess of the fixed element (12) is electrically connected to the electrical circuit (3) of the control unit (1) through the second set of electrical tracks (14), wherein the electrical tracks on the periphery of the recess take the form of rings arrayed in different transverse planes.

17. The adjustable patch device according to any of Claims 12 to 16, **characterized in that** the fitting of the protrusion of the rotating element (11) into the recess of the fixed element (12) leads to electrical connection between the rings of the first set of electrical tracks (13) and the rings of the second set of electrical tracks (14) along different transverse planes, wherein this electrical connection is maintained during the rotational movement of the rotating element (11) on the fixed element (12).

18. The adjustable patch device according to any of the previous Claims, **characterized in that** it is attached to the user's body by means of a mechanism selected from the group consisting of adhesives, gel electrodes (16), or suction mechanisms.

19. The adjustable patch device according to any of the previous Claims, **characterized in that** at least one of the physiological data collection elements (3) comprises a plurality of electrodes or sensors arrayed in proximity to each other.

20. The adjustable patch device according to Claim 19, **characterized in that** the electrodes are gel electrodes (16) or dry electrodes (17), wherein said electrodes are configured to collect electrical signals obtained through techniques selected from the group consisting of electrocardiography, electromyography, electrogastrography, electrodermal activity, electroencephalography, body temperature, and electrooculography.

21. The adjustable patch device according to Claim 19, **characterized in that** the sensors are selected from the group consisting of oximetry sensors or spectroscopy sensors.

22. The adjustable patch device according to any of the previous Claims, **characterized in that** it comprises additional sensors based on electromagnetic or mechanical waves that are not in contact with the user's body.
